**Europäisches Patentamt**

(19) **European Patent Office**

**Office européen des brevets**

(11) Publication number: **0 109 012**
A2

---

(12) # EUROPEAN PATENT APPLICATION

(21) Application number: **83110996.2**

(22) Date of filing: **04.11.83**

(51) Int. Cl.³: **G 01 N 33/54**

---

(30) Priority: **12.11.82 US 441025**

(43) Date of publication of application: **23.05.84**
**Bulletin 84/21**

(84) Designated Contracting States: **BE DE FR GB IT**

(71) Applicant: **ABBOTT LABORATORIES, 14th Street and Sheridan Road, North Chicago, Illinois 60064 (US)**

(72) Inventor: **Firca, Joseph Ronald, 290 Sierra Oaks Drive, Arcadia California (US)**
Inventor: **Knigge, Kevin Michael, 1166 Northbury Lane, Wheeling Illinois (US)**
Inventor: **Babb, James Lowell, 730 Crane Boulevard, Libertyville Illinois (US)**

(74) Representative: **Modiano, Guido et al, MODIANO, JOSIF, PISANTY & STAUB Modiano & Associati Via Meravigli, 16, I-20123 Milan (IT)**

---

(54) **Determination of streptococci.**

(57) The present invention relates to an improved immunoassay for determining streptococcal antigen in a clinical specimen. In particular, the present invention relates to a solid phase immunoassay for directly determining streptococcal antigen.

EP 0 109 012 A2

## Background Of The Invention

*Streptococci* are a ubiquitous group of gram positive cocci which tend to grow in chains. The *Streptococci* may be differentiated into three broad categories according to their hemolytic characteristics on blood agar medium; alpha (partial or "green" hemolysis), beta (complete hemolysis) and gamma (no hemolysis). Beta-hemolytic pathogenic *Streptococci* of Lancefield's Group A are responsible for most human streptococcal infections. Although the exact incidence of streptococcal pharyngitis is unknown, it is one of the most frequent bacterial infections of humans. The incidence is highest in children, males and females being equally affected. If antibiotics are not administered, patients may develop serious poststreptococcal sequelae such as acute rheumatic fever and acute glomerulonephritis. Wannamaker, *Reviews of Infectious Disease*, 1:967-973 (1979), Catanzaro, et al, *American Journal of Medicine*, 749-756, (1954).

The current method of diagnosis involves isolation of *Streptococci pyogenes* from throat swabs cultured on sheep blood agar, Facklam, *Isolation and Identification of Streptococci*, CDC Publication (1980). For presumptive identification a paper disc impregnated with bacitracin is placed on the sheep blood agar in the area of heaviest inoculation. After incubation at 37° C for 24 hours, *Streptococcus pyogenes* will exhibit complete hemolysis with a zone of inhibition around the bacitracin disc. Stoner, *J. Clin. Microbiol.*, 7:463-466 (1978), Pollock, et al, *Appl. Microbiol.* 27:141-143 (1979). For definitive identification of *Streptococcus pyrogenes*, laboratories use fluorescent antibody staining, Ederer, et al, *Appl. Microbiol.* 24:160-161 (1972) latex agglutination Watson, et al, *J. Clin. Microbiol*, 1:268-273 (1975), coagglutination, Stoner, *supra*, Rosner, *J. Clin. Microbiol.*, 6:23-26 (1977) or Lancefield typing, Pollock, et al, *Appl. Microbiol*, 27:141-143 (1974).

U. S. Patent No. 4,329,151 describes a method for qualitatively determining *Streptococci* infections employing agglutination techniques utilizing streptolysin-0-protein directly absorbed onto polystyrene latex particles. U. S.

Patent No. 3,790,447 describes a method for detecting streptococcal organisms comprising a smear of the organism being placed on a microscopic slide and is treated with antibody conjugated to peroxidase, and the slide is examined under a microscope after drying with enzyme substrates to determining the presence of Group A *Streptococci*.

It is known that various substances, commonly referred to as "blocking factors" will inhibit antibody-antigen interactions by "blocking" the binding sites on the antigen. Such blocking substances or blocking factors are generally a coating of host antibody against surface antigens. It has further been reported that most human sera contains blocking substances which adhere tightly to Group A *Streptococci* and prevent staining with fluorescent antistreptococcal antibody. Such blocking substances will bind to the antigen to be determined, and thereby decrease the sensitivity of immunoassays for Group A *Streptococci*.

The present invention relates to a method for determining *Streptococci* infection in a sample utilizing enzymeimmunoassay techniques wherein the effect of blocking factors is minimized.

## Summary Of The Invention

The present invention relates to an improved immunoassay procedure for determining streptococcal antigen in a clinical specimen. A sample suspected of containing streptococcal antigen is treated with an effective amount of a composition comprising a lytic enzyme and a surfactant. The treated sample is contacted with an antibody to *Streptococci* to bind group specific antigen and form a suspension containing an antibody-group specific antigen complex. The antibody-group specific antigen suspension is then contacted with an antigen coated on a solid support. Any uncomplexed antibody to *Streptococci* will bind to the antigen coated on the solid support. Unbound material is removed and the antigen-antibody complex on the solid support is treated with anti Strep to form an antigen-antibody-anti Strep complex on the solid support. The amount of anti Strep bound to the antigen-antibody complex is measured as an indication of the amount of streptococcal antigen in the

## Detailed Description Of The Invention

In accordance with the method of the present invention, a clinical specimen, containing the streptococcal antigen to be detected, is obtained from a patient utilizing conventional medical and microbiological techniques.  Such clinical specimens include, for example, swab specimens obtained from the throat or skin of the patient, as well as urine, blood, saliva, cerebrial spinal fluid specimens obtained from the patient.  If a swab specimen is assayed, it is maintained in a dry condition prior to analysis.

In order to maximize exposure of group specific streptococcal antigen and to inhibit nonspecific absorption of protein, the sample is treated with an effective amount of the composition comprising a lytic enzyme and surfactant. The lytic enzymes effective in the compositions of the present invention are readily ascertained by one of ordinary skill in the art and include for example, mutanolysin.  The surfactant effective in the compositions of the present invention include, for example, sodium lauroyl sarcosinate. An "effective amount" of the compositions comprising a lytic enzyme and surfactant refer to the amount of lytic enzyme and surfactant required to lyse the *Streptococci* organism and provide maximum exposure of group specific streptococcal antigen while inhibiting interferences due to blocking substances.  The preferred compositions comprise approximately 75 µg/ml of mutanolysin and 0.1% sodium lauroyl sarcosinate in a borate buffer (pH 6.5).

Representative of the streptococcal antigen that may be determined by the methods of the present invention include, for example, Group A *Streptococci (S. pyogenes)*; Group B *Streptococci (S. agalactiae)*; Group C *Streptococci (S. equisimilis)* and the like.

According to a preferred embodiment of the present invention, the treated sample containing the streptococcal antigen to be assayed is treated with antibody to *Streptococci* to form a suspension containing a group specific antigen-antibody complex.  The treated sample containing the streptococcal antigen and the antibody to *Streptococci* is incubated for a sufficient period of time to permit maximum formation of a group specific antigen-antibody complex. Upon completion of the incubation, an aliquot of the suspension containing unbound antibody to *Streptococci* and a group specific antigen-antibody complex, is incubated with an antigen coated on a solid support, wherein the antigen is specific for the antibody to *Streptococci*. Any unbound antibody to *Streptococci* will bind to the antigen coated on the solid support forming an antigen-antibody complex on the solid support. The greater the number of streptococcal organisms present in the treated sample, the lower the amount of unbound antibody to *Streptococci* will be available for binding to the antigen coated solid support. Unbound material is removed and the antigen-antibody complex coated on the solid support is washed and then treated with anti Strep. The amount of anti Strep bound to the antigen-antibody complex is measured as an indication of the amount of streptococcal antigen in the sample.

Solid support refers to an insoluble polymeric material sorptive for streptococcal antigen. Known material of this type include hydrocarbon polymers such as polystyrene, polyethylene, polypropylene, polybutylene, butyl rubber and other synthetic rubbers. Other suitable organic polymers include salastic rubber, polyesters, polyamides, cellulose and cellulosic derivatives, acrylate, methylacrylates, vinyl chloride, and polyvinyl chloride. Copolymers such as graph copolymers of polystyrene are also useful. In addition to the foregoing materials, the solid surface may comprise silica gel, silica waffers, glass insoluble protein metals, and the solid support may be in the form of beads, tubes, strips and discs and the like.

The term "antibody to *Streptococci*" refers to an antibody that is directed to or specific to the particular group specific streptococcal antigen to be determined and is raised in a nonhuman species such as rabbit, goat, horse, sheep, guinea pig, etc. For example, if the group specific streptococcal antigen to be determined is Group A *Streptococci*, (*S. pyogenes*), the antibody to *Streptococci* employed is antibody to *S. pyogenes*. The antibodies to *Streptococci* effective in the methods of the present invention are produced employing strains of the particular group specific *Streptococci* as immunogens in accordance with known techniques.

As used herein the term "anti Strep antibody" refers to an antibody directed or specific for antibody to *Streptococci*, and is raised in a nonhuman species such as rabbit, goat, horse, sheep, guinea pig, etc. As previously mentioned, the antigen-antibody complex is reacted with an anti Strep to form an antigen-antibody-anti Strep complex on the solid support and the complex is determined as a measure of the concentration of streptococcal antigen in the sample. The anti Strep may be directly labeled with a label such as an enzyme or a fluorescent tag, such as a fluorescent dye to permit determination of the amount bound, or may be indirectly labeled by further reaction, for example, with an antibody specific for anti Strep which is labeled with an enzyme, etc., by conventional methods.

It is preferred to employ direct enzyme labelling of the anti Strep. Examples of enzymes include catalase, peroxidase, urease, glucose oxidase, phosphatase, and the like. If direct labelling of the anti Strep is employed, following the formation of the antigen-antibody-anti Strep* complex, wherein anti Strep* refers to labeled anti Strep, an enzyme substrate is added to the ligand and/or solid support of the reaction mixture and the enzymatic determination is performed utilizing conventional techniques such as colorimetric determinations to measure bound labeled anti Strep. In the case of indirect labelling, that is, the anti Strep in unlabelled, the antigen-antibody-anti Strep complex is washed to remove unbound anti Strep and subsequently reacted with a labeled antibody to the anti Strep and the bound antibody is then measured.

The following examples illustrate the present invention and are not intended to limit it in spirit or in scope.

## EXAMPLE 1
## PREPARATION OF ANTIGEN COATED BEADS

Streptococcal antigen was isolated from *Streptococci* cultures and was conjugated to protein. The protein conjugate was diluted 1:5000 in 0.1M borate buffer (pH 9.3). The diluted solution was used to coat 6mm polystyrene beads overnight at room temperature and each set of beads was washed and air dried.

## DETERMINATION OF *STREPTOCOCCUS PYOGENES*

1. A throat swab was placed in a tube to which was added 200 $\mu\ell$ of a 0.001M phosphate buffer (pH 6.5) containing 0.1% sodium lauroyl sarcosinate. To the tube containing the swab and the tubes containing 200 $\mu\ell$ of positive and negative controls was then added 200 $\mu\ell$ of a lytic composition containing 75 $\mu$g/ml of mutanolysin in distilled water. The swab was swirled vigorously for 10 seconds after which time the tubes were incubated in a water bath at 37° C for 28-32 minutes.

2. Following the incubation period, 200 $\mu\ell$ of a solution containing antibody to *S. pyogenes*, 50% fetal calf serum in 0.05M Tris buffer (pH 7.2) was added to each tube. The tubes were incubated in a water bath at 37° C for 18-32 minutes.

3. Following the incubation period, the tubes were removed from the water bath and excess fluid was expressed from the swab by pressing and rotating the swab against the side of the tube and the swab was then discarded.

4.  A 200 µℓ aliquot of an extracted sample containing *S. pyogenes* and of the controls was added to appropriate wells of the reaction tray.

5.  A polystyrene bead coated with *S. pyogenes* antigen was added to each well containing a sample or controls and the reaction trays are covered and incubated at 37° C in a water bath for 18-22 minutes.

6.  Following the incubation period, unbound sample or controls were removed from the wells and the beads were washed three times with water.

7.  To the wells containing the washed beads was added 200 µℓ of a solution containing from 2 µg/ml of antibody to rabbit IgG (goat) covalently linked to horseradish peroxidase in 45% fetal serum,  5% normal human serum in 0.05M Tris buffer (pH 7.2).

8.  The reaction trays were covered and incubated at 37° C in a water bath for 18-22 minutes.

9.  Following the incubation, unbound antibody to rabbit IgG (goat)-horseradish peroxidase conjugate was removed and the beads were washed three times with water.

10.  The beads from the wells originally containing the samples and controls were transferred to assay tubes to which was then added 300 µℓ of a freshly prepared substrate solution containing approximately 27 mg of σ-phenylene diamine·2HCl in 5 ml of citrate-phosphate buffer containing 0.02% hydrogen peroxidase at a pH of 5.5.  The tubes were then incubated for 9-11 minutes at room temperature.

11. Following the incubation, 1 ml of 1N sulfuric acid was added to each tube and the absorbance of the resulting sample and control solutions were read on a spectrophotometer at 492 nm.

12. The absorbance value for the sample is compared to the absorbance value for positive and negative controls to determine the presence of streptococcal antigen.

Assays for Group A *Streptococci (S. pyogenes)* were conducted in accordance with the procedure described in Example I, with and without the use of a surfactant in the pretreatment of the sample (Step 1). It was noted that the sensitivity and specificity of the assay significantly increased due to the use of a surfactant, in particular, sodium lauroyl sarconsinate, in conjunction with a lytic enzyme, in particular, mutanolysin, in the pretreatment (Step 1) of the sample to be assayed.

Although this invention has been described with respect to specific modification, the details thereof are not to be construed as limitations, for it will be apparent that various equivalents, changes and modifications may be resorted to without departing from the spirit and scope thereof and it is understood that such equivalent embodiments are intended to be included therein.

0109012

## CLAIMS

1. A method for determining streptococcal antigen in a clinical sample comprising:

a) treating the sample with an effective amount of a lytic enzyme and a surfactant in a buffered medium;

b) contacting the treated sample with antibody to *Streptococci* to form a suspension containing an unbound antibody to *Streptococci* and an antibody-group specific antigen complex;

c) contacting the suspension containing the unbound antibody to *Streptococci* with an antigen coated solid support wherein the antigen is specific for the antigen to *Streptococci* to form an antigen-antibody complex on the solid support;

d) removing unbound materials;

e) treating the antigen-antibody complex on the solid support with anti Strep to form an antigen-antibody-anti Strep complex on the solid support;

f) separating the antigen-antibody-anti Strep complex on the coated solid support from unbound anti Strep; and

g) determining the antigen-antibody-anti Strep complex on the solid support as a measure of the streptococcal antigen in the sample.

2. A method according to Claim 1 wherein the lytic enzyme is mutanolysin and the surfactant is sodium lauroyl sarcosinate.

3. A method according to Claim 2 wherein the anti Strep is labeled with an enzyme.

4. A method according to Claim 3 wherein the streptococcal antigen to be determined is Group A *Streptococci*.

5. A method as in any of Claims 1-4 wherein the sample is Step (a) is treated first with a surfactant in a buffered medium and then with a lytic enzyme.